# EUROPEAN PATENT APPLICATION

(11) **EP 3 510 943 A1**
(43) Date of publication of application: **17.07.2019**
(21) Application number: 17856233.6
(22) Date of filing: 27.09.2017
(51) Int. Cl.: A61B 17/06, A61B 17/42

(54) **SUTURE NEEDLE FOR UTERINE HEMOSTATIC COMPRESSION**

(30) Priority: 29.09.2016 JP 2016190970
(71) Applicant: Alfresa Pharma Corporation, Osaka-shi, Osaka 540-8575 (JP); Osaka University, Suita-shi, Osaka 565-0871 (JP)
(72) Inventor: SHIONO, Toshimi, Osaka-shi Osaka 540-8575 (JP); FURUTA, Shinji, Osaka-shi Osaka 540-8575 (JP); MATSUZAKI, Shinya, Suita-shi Osaka 565-0871 (JP); KIMURA, Tadashi, Suita-shi Osaka 565-0871 (JP); ENDO, Masayuki, Suita-shi Osaka 565-0871 (JP)
(74) Representative: Müller-Boré & Partner Patentanwälte PartG mbB
(86) International application number: PCT/JP2017/034988
(87) International publication number: WO 2018/062284

(57) **Abstract**

A suture needle for uterine hemostatic compression (1) includes a suture needle (10) and a suture thread (13). The suture needle (10) includes a body portion (14) extending linearly in a lengthwise direction (D1) and a puncture portion (15) provided at a tip of the body portion (14) in the lengthwise direction (D1) to be thrust into a uterus. The suture thread (13) is attached to a base end portion (17) that is on an opposite side of the puncture portion (15) in the suture needle (10). The puncture portion (15) is formed as a spherical surface that bulges toward a tip side of the lengthwise direction (D1) and has a radius in a connection portion with the body portion (14) substantially identical to a radius of the body portion (14).

## Description

### Technical Field

The present invention relates to a suture needle for uterine hemostatic compression.

### Background Art

Conventionally, as disclosed in Patent Literature 1 to be described below, various types of suture needle used for suturing organ tissues in surgical operations are known. Suturing in surgical operations is performed by an operator threading a suture thread by thrusting a needle tip into a target organ while holding a suture needle with a needle holder or a hand and ligating the organ with the suture thread. Under the current circumstances, various types of suture needle are used as appropriate according to a type, properties, and the like of the internal organ to be sutured. Patent Literature 1 below discloses a suture needle having a shape where the overall needle is curved in a semicircular shape and the needle tip is pointed.

During delivery at an obstetrician clinic, atonic bleeding that causes massive bleeding resulting from a fact that a uterine muscle does not have a good contraction after expulsion of a baby is observed in about 5-percent cases. There is also a disease called a placenta previa that disables a vaginal delivery and needs a caesarean section because the placenta covers the uterine os. This placenta previa is the most dangerous disease in delivery, and an average of 1500 mL of bleeding is observed, which is about three times as heavy as normal delivery. In many of these diseases, a manipulation to perform hemostatic compression by thrusting a suture needle into a part called a body lower portion of the uterus and ligating the body lower portion with a suture thread is effective. However, the manipulation is currently not widely practiced due to the difficulty of the manipulation and the lack of dedicated suture needle.

Here, since there are many blood vessels in neighboring tissues of the body lower portion of the uterus, if a conventional suture needle as disclosed in Patent Literature 1 is used, the suture needle may damage the blood vessels of the neighboring tissues when the suture needle is thrust into the body lower portion. Also, since the body lower portion of the uterus is close to peripheral organs such as the bladder and the rectum, suturing the body lower portion of the uterus has a high risk of organ damage. This makes it difficult to suture an area effective for hemostatic compression. That is, blood vessels or peripheral organs are damaged in some cases by the sharply pointed needle tip, and there is a problem that it is difficult to safely perform hemostasis of atonic bleeding.

### Citation List

### Patent Literature

Patent Literature 1: Japanese Patent Application Laid-Open No. H08-52145

### Summary of Invention

It is an object of the present invention to provide a suture needle for uterine hemostatic compression that enables an operation to perform uterine hemostatic compression to be safely performed.

A suture needle for uterine hemostatic compression according to one aspect of the present invention includes at least one suture needle and a suture thread. The at least one suture needle includes a body portion extending linearly in a lengthwise direction and a puncture portion provided at a tip of the body portion in the lengthwise direction to be thrust into a uterus. The suture thread is attached to a base end portion that is on the opposite side of the puncture portion in the suture needle. The puncture portion is formed as a spherical surface that bulges toward a tip side of the lengthwise direction, the spherical surface having a radius in a connection portion with the body portion substantially identical to a radius of the body portion.

### Brief Description of Drawings

FIG. 1 is a schematic view showing a configuration of a suture needle for uterine hemostatic compression according to a first embodiment of the present invention.
FIG. 2 is an enlarged view of the suture needle for uterine hemostatic compression in an area II of FIG. 1.
FIG. 3 is a schematic view for describing a manipulation using the suture needle for uterine hemostatic compression.
FIG. 4 is a schematic view for describing a method of hemostatic compression suture of a uterine body (hereinafter, compression suture).
FIG. 5 is a schematic view showing how a suture thread passes through a uterine front wall below a caesarean section wound portion from outside of the uterus to inside of the uterus (left side).
FIG. 6 is a schematic view showing how the suture thread is pulled out of the uterine front wall above the caesarean section wound portion from inside of the uterus to outside of the uterus (left side).
FIG. 7 is a schematic view showing how the suture thread goes around from the uterine front wall toward a uterine rear wall (left side).
FIG. 8 is a schematic view showing how the suture thread passes through the uterine rear wall from outside of the uterus to inside of the uterus (left side).
FIG. 9 is a schematic view showing how the suture thread is pulled out of the uterine rear wall from inside of the uterus to outside of the uterus (right side).
FIG. 10 is a schematic view showing how the suture thread goes around from the uterine rear wall toward the uterine front wall (right side).
FIG. 11 is a schematic view showing how the suture thread passes through the uterine front wall above the caesarean section wound portion from outside of the uterus to inside of the uterus (right side).
FIG. 12 is a schematic view showing how the suture thread is pulled out of the uterine front wall below the caesarean section wound portion from inside of the uterus to outside of the uterus (right side).
FIG. 13 is a schematic view for describing one example of suturing a uterine body lower portion.
FIG. 14 is a schematic view for describing another example of suturing the uterine body lower portion.
FIG. 15 is a schematic view for describing still another example of suturing the uterine body lower portion.
FIG. 16 is a schematic view showing a configuration of a suture needle for uterine hemostatic compression according to another embodiment of the present invention.
FIG. 17 is a schematic view showing a configuration of a suture needle for uterine hemostatic compression according to another embodiment of the present invention.
FIG. 18 is a schematic view showing a configuration of a suture needle for uterine hemostatic compression according to another embodiment of the present invention.

### Description of Embodiments

Embodiments of the present invention will be described in detail below with reference to the drawings.

### (First Embodiment)

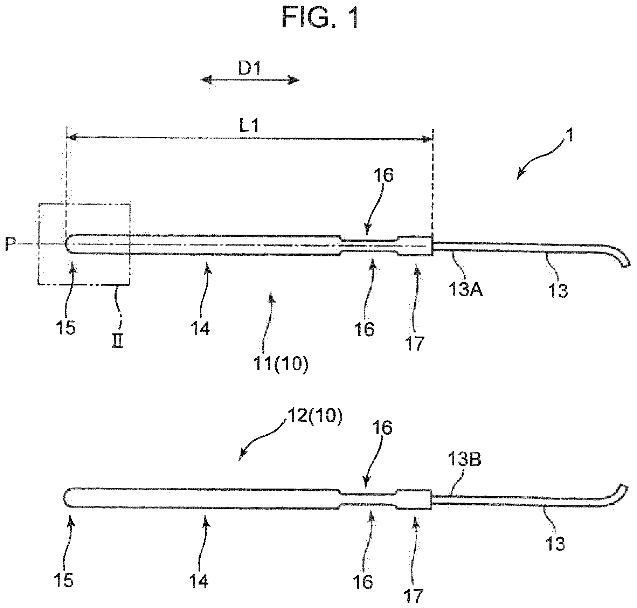

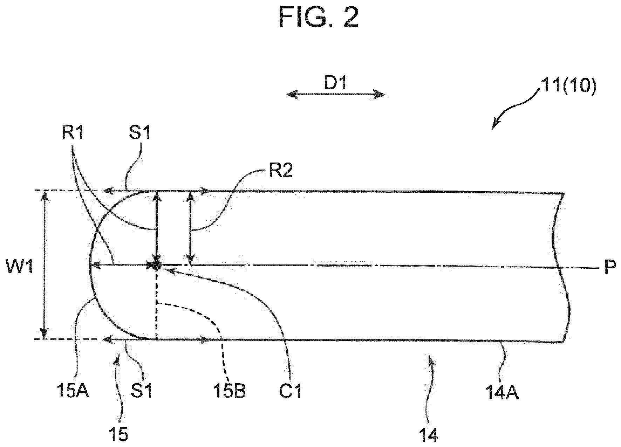

First, a configuration of a suture needle for uterine hemostatic compression 1 according to a first embodiment of the present invention will be described with reference to FIG. 1 and FIG. 2. FIG. 1 is a view schematically showing the overall configuration of the suture needle for uterine hemostatic compression 1. FIG. 2 is a view showing an enlarged configuration of a tip portion of the suture needle for uterine hemostatic compression 1.

The suture needle for uterine hemostatic compression 1 is used for a manipulation to perform uterine hemostatic compression such as medical treatment of atonic bleeding. The atonic bleeding is a case that occurs during delivery at an obstetrician clinic, and is a disease that causes massive bleeding resulting from a fact that a uterine muscle does not have a good contraction after expulsion of a baby. Note that the suture needle for uterine hemostatic compression 1 is used not only for the case of atonic bleeding, but also for hemostasis in a placenta previa (disease of formation of a placenta in a lower portion of the uterus), for example.

As shown in FIG. 1, the suture needle for uterine hemostatic compression 1 includes a first suture needle 11, a second suture needle 12 and a suture thread 13 that connects these suture needles to each other. Note that a middle portion of the suture thread 13 is omitted in FIG. 1 for convenience of drawing, but the first suture needle 11 and the second suture needle 12 are connected to each other by one suture thread 13. Since the first suture needle 11 and the second suture needle 12 are the same in the present embodiment, only the configuration of the first suture needle 11 will be described in detail, and a detailed description of the second suture needle 12 will be omitted. Note that in the following descriptions, the first suture needle 11 and the second suture needle 12 are also simply called "suture needle 10."

The suture needle 10 includes a body portion 14 extending linearly in a lengthwise direction D1, a puncture portion 15 provided at a tip of the body portion 14 in the lengthwise direction D1, a grip portion 16 provided on an opposite side of the body portion 14 from the puncture portion 15, the grip portion 16 being a portion for an operator to hold the suture needle 10, and a base end portion 17 provided on an opposite side of the grip portion 16 from the body portion 14, the base end portion 17 being a portion to which an end of the suture thread 13 is attached. As shown in FIG. 1, the suture needle 10 has a shape extending linearly in the lengthwise direction D1 by the puncture portion 15, the body portion 14, the grip portion 16 and the base end portion 17 being connected in order from the tip side. That is, the suture needle 10 does not have a curved shape but has a straight axis line P extending in parallel to the lengthwise direction D1. Also, in any part of the lengthwise direction D1, the thickness of the suture needle 10 is between 0.5 mm and 1.5 mm inclusive such that an operator can bend the suture needle 10 easily for use.

The puncture portion 15 is a part to be thrust into the uterus during an operation. The suture needle for uterine hemostatic compression 1 according to the present embodiment has a characteristic that the puncture portion 15 is formed as a spherical surface. As shown in FIG. 2, a spherical surface 15A is formed to bulge toward the tip side of the lengthwise direction D1 and to make a radius R1 in a connection portion with the body portion 14 substantially identical to a radius R2 of the body portion 14. More specifically, the puncture portion 15 is configured as a hemispheric body having the radius R1, and is connected to an end of the body portion 14 at a terminal 15B of the spherical surface 15A. That is, the spherical surface 15A is a hemispherical surface. Therefore, as shown in FIG. 2, the center C1 of the spherical surface 15A is positioned at the terminal 15B of the spherical surface 15A.

In this way, the suture needle 10 is an extremely dull needle with the puncture portion 15 to be thrust into the uterus having a shape that is not sharply pointed but a round shape with a spherical surface. Therefore, when thrusting the suture needle 10 into the body lower portion of the uterus for penetration during the operation, it is possible to prevent blood vessels from being damaged by contact with the puncture portion 15 in neighboring tissues of the body lower portion of the uterus.

The radius R1 of the spherical surface 15A is 0.5 mm or more. In this case, compared with a case where the radius R1 is less than 0.5 mm, thrusting resistance of the suture needle 10 (force necessary for thrusting the suture needle 10 into an object) increases significantly. Therefore, by setting the radius R1 at 0.5 mm or more, it is possible to more securely prevent blood vessels in neighboring tissues of the body lower portion of the uterus from being damaged when the suture needle 10 is thrust. In this way, from a viewpoint of preventing blood vessel damage in the body lower portion of the uterus, the radius R1 is more preferably 0.55 mm or more.

The width W1 of the puncture portion 15 is 1 mm or more. As shown in FIG. 2, the width W1 is the width of the suture needle 10 at the terminal 15B of the spherical surface 15A. This width W1 is a diameter (R1 × 2) of the spherical surface 15A and corresponds to an outer diameter of the body portion 14.

When the width W1 is 1 mm or more, the thrusting resistance of the suture needle 10 greatly increases as compared with a case where the width W1 is less than 1 mm. Therefore, by setting the width W1 of the puncture portion 15 at 1 mm or more, it is possible to more securely prevent the blood vessels in neighboring tissues of the body lower portion of the uterus from being damaged as described above. In this way, from a viewpoint of preventing blood vessel damage in the body lower portion of the uterus, the width W1 is preferably 1.05 mm or more, more preferably 1.1 mm or more, and even more preferably 1.15 mm or more. Note that the radius R1 and the width W1 are preferably within the above ranges, but are not particularly limited.

The body portion 14 has a cylindrical shape and is formed so as to extend from the terminal 15B of the spherical surface 15A toward the base end portion 17 in the lengthwise direction D1. As shown in FIG. 2, the body portion 14 has the same width (outside diameter of the cylinder) as the width W1 (R1 × 2) of the puncture portion 15, and straightly extends in the lengthwise direction D1 while keeping the constant width. An outer peripheral surface 14A of the body portion 14 is formed so as to follow a tangent line S1 at the terminal 15B of the spherical surface 15A. The outer peripheral surface 14A and the tangent line S1 both extend in parallel to the lengthwise direction D1 of the suture needle 10.

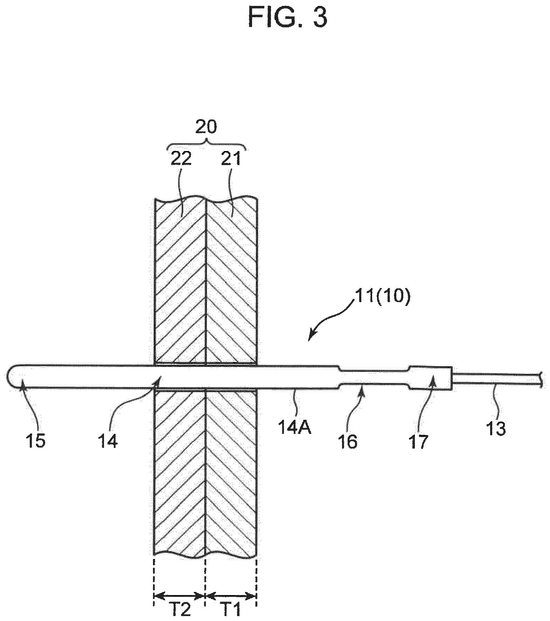

For this reason, as shown in FIG. 3, when thrusting the suture needle 10 into the uterus 20 (front wall 21 and rear wall 22), it is possible to cause the body portion 14 to travel straight along a path through which the puncture portion 15 passes. Therefore, unlike a case of using the suture needle having a shape with the body portion 14 being curved, it is possible for the operator to securely suture a part to be sutured, and to minimize a risk of damaging blood vessels of neighboring tissues of the uterus 20 by mistake. Note that as will be described later, the suture needle for uterine hemostatic compression of the present invention is not limited to the needle with the body portion 14 having a constant width (outside diameter) in this way.

The grip portion 16 is a portion for the operator to hold the suture needle 10 with a needle holder or a hand. As shown in FIG. 1, the grip portion 16 is formed to be recessed radially inward from the body portion 14 and the base end portion 17 to allow the operator to easily hold the grip portion 16. Therefore, the grip portion 16 is smaller (thinner) than the body portion 14 and the base end portion 17 in outside diameter. The operator can easily hold the suture needle 10 in the grip portion 16 and thrust the puncture portion 15 into the uterus while holding the suture needle 10 in the grip portion 16. However, the grip portion 16 is not an essential component in the suture needle for uterine hemostatic compression of the present invention, and the grip portion 16 may be omitted.

The base end portion 17 is provided at a base end of the suture needle 10 in the lengthwise direction D1 (opposite side of the puncture portion 15). A (unillustrated) hole for inserting the end of the suture thread 13 is formed in the base end portion 17. Then, the suture thread 13 is fixed to the base end portion 17 by putting the end of the suture thread 13 into the hole and caulking the end. Note that some suture thread 13 is fixed to the base end portion 17 with force of a level that allows the suture thread 13 to be detached by the operator pulling after the manipulation is finished, but the suture thread 13 is not limited to this example. Also, a method of fixing the suture thread 13 is not limited to this example, and for example, another method such as winding the suture thread 13 around the base end portion 17 may be used.

The total length L1 of the suture needle 10 in the lengthwise direction D1 (length from the puncture portion 15 to the base end portion 17) is between 60 mm and 120 mm inclusive. As shown in FIG. 3, in hemostasis of atonic bleeding, the uterus 20 is pushed and contracted in a front and rear direction such that the front wall 21 and the rear wall 22 come into contact with each other, the suture needle 10 performs penetration at a stretch from the front wall 21 toward the rear wall 22, and the operator checks the puncture portion 15 protruding from the rear wall 22 by touching the puncture portion 15 with a finger. Therefore, the suture needle 10 needs enough length L1 to perform such a manipulation.

Thicknesses T1 and T2 of the front wall 21 and the rear wall 22 are about 25 mm during non-pregnancy. The thicknesses T1 and T2 in the pregnant uterus after expulsion of a baby is about total 50 mm when pressed with fingers. When a length of a portion in which the operator holds the suture needle 10 on the front wall 21 side and a length of a portion protruding from the rear wall 22 are taken into consideration, the length L1 of the suture needle 10 is preferably 60 mm or more, more preferably 80 mm or more, and even more preferably 100 mm or more. However, since the handling of the suture needle 10 will worsen when the length L1 is too long, the length L1 is preferably 120 mm or less.

The suture thread 13 is used for hemostatic compression by sewing up the uterus 20. As shown in FIG. 1, one end 13A of the suture thread 13 is attached to the base end portion 17 of the first suture needle 11, and the other end 13B is attached to the base end portion 17 of the second suture needle 12.

As the suture thread 13, for example, a thread made of a synthetic absorbent material such as polydioxanone and polyglycolic acid can be used. These materials, which melt in vivo, are particularly preferable in terms of biocompatibility. However, the material of the suture thread 13 is not limited to these examples. For example, a thread made of a material such as silk, which is a natural unabsorbent material, a synthetic unabsorbent material including nylon, polypropylene, polyvinylidene fluoride, polyester or polyethylene can also be used.

The suture thread 13 has a length of 50 cm or more from a viewpoint of a sufficient length for sewing up the uterus 20 in hemostasis of atonic bleeding. Also, the suture thread 13 is thinner than the suture needle 10, and for example has a thickness between 0.5 mm and 0.599 mm inclusive.

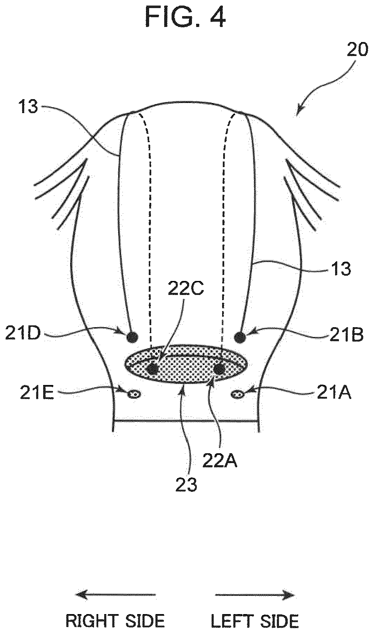

Next, one example of hemostatic methods of atonic bleeding using the suture needle for uterine hemostatic compression 1 will be described. These are methods for the purpose of uterine body hemostatic compression. First, by compression suture to be described below, as shown in FIG. 4, the uterus 20 is contracted vertically using the suture thread 13. Note that notation of "right side" and "left side" in FIG. 4 indicates directions on the basis of a patient.

Basically, compression suture is started before suturing the caesarean section wound portion 23.

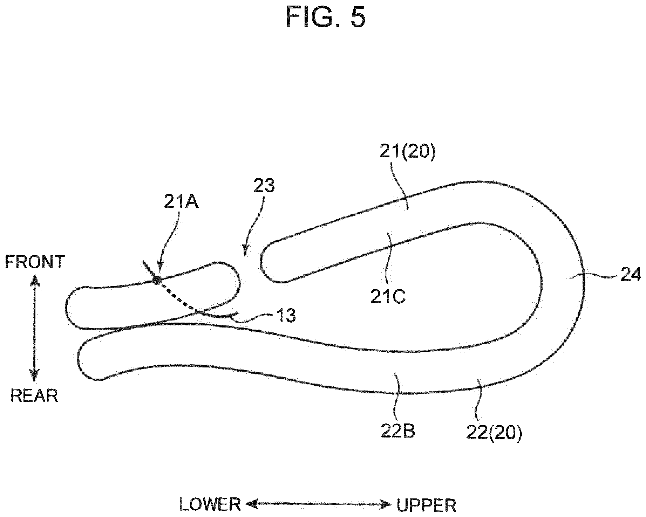

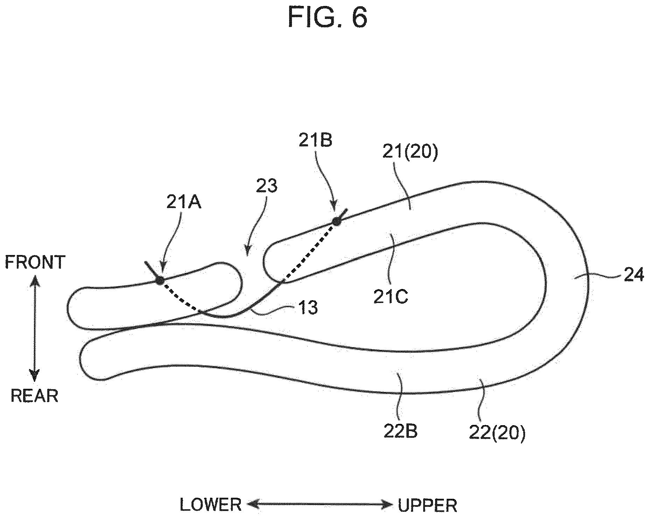

FIG. 5 to FIG. 8 sequentially show how the uterus 20 is ligated with the suture thread 13 in a left portion of FIG. 4. First, the operator presses the uterus 20 strongly in a front and rear direction with both hands to confirm that bleeding in the vagina direction stops. After this confirmation, first, the suture thread 13 is passed inward of the uterus 20 by causing the suture needle to penetrate a front wall portion 21 below the caesarean section wound portion 23 from outside of the uterus toward inside of the uterus (FIG. 5). Next, the suture thread 13 is pulled out from the front wall 21 side to outside of the uterus by causing the suture needle to penetrate the front wall portion 21B above the caesarean section wound portion 23 from inside of the uterus toward outside of the uterus (FIG. 6).

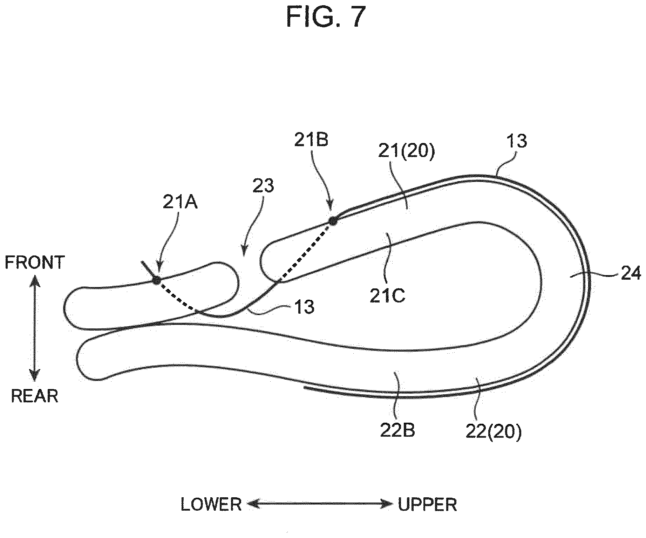

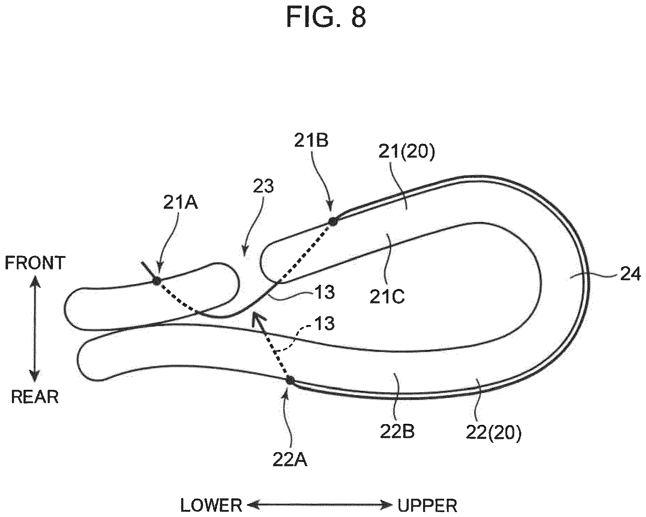

Next, the suture thread 13 goes around a body part 21C of the front wall 21, a bottom portion 24 and a body part 22B of the rear wall 22 in this order (FIG. 7). Then, the suture thread 13 is again passed inward of the uterus 20 by causing the suture needle to penetrate a rear wall portion 22A corresponding to the height of the caesarean section wound portion 23 from outside of the uterus toward inside of the uterus (FIG. 8). In this procedure, the left part of the uterus 20 is ligated with the suture thread 13 as shown in FIG. 4.

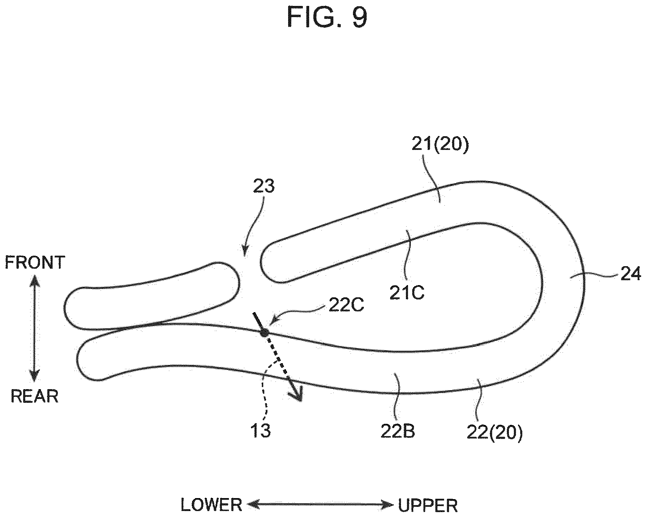

Next, the right part of the uterus 20 is similarly ligated with the suture thread 13. FIG. 9 to FIG. 12 sequentially show how the uterus 20 is ligated with the suture thread 13 in a right portion of FIG. 4. First, the suture thread 13 is pulled out from the rear wall 22 side to outside of the uterus by causing the suture needle to penetrate the rear wall portion 22C corresponding to the same height as the caesarean section wound portion 23 from inside of the uterus toward outside of the uterus (FIG. 9).

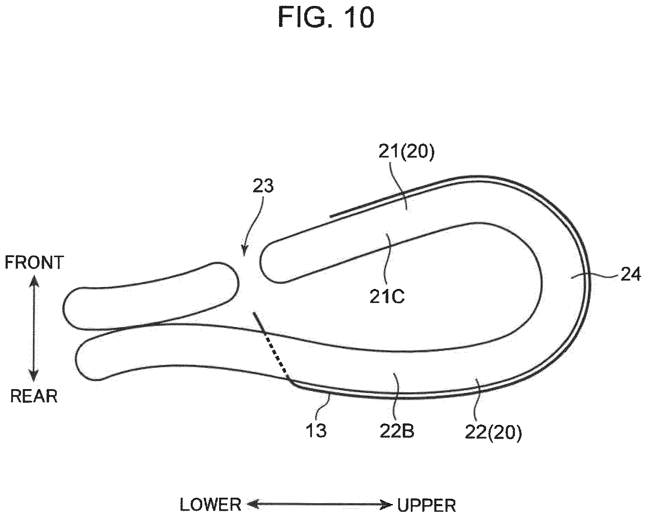

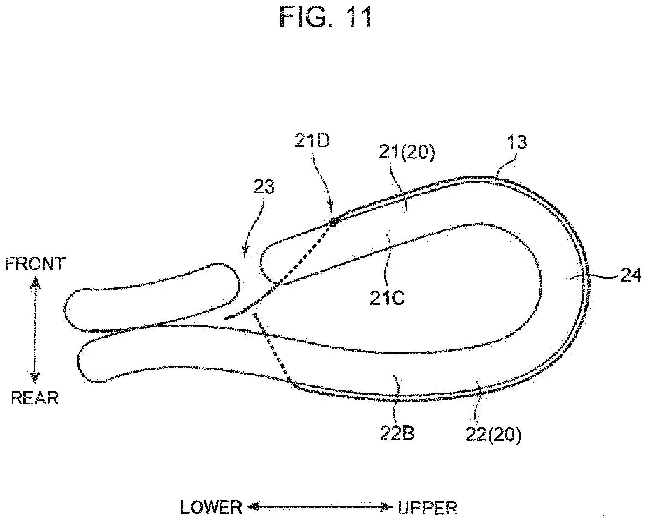

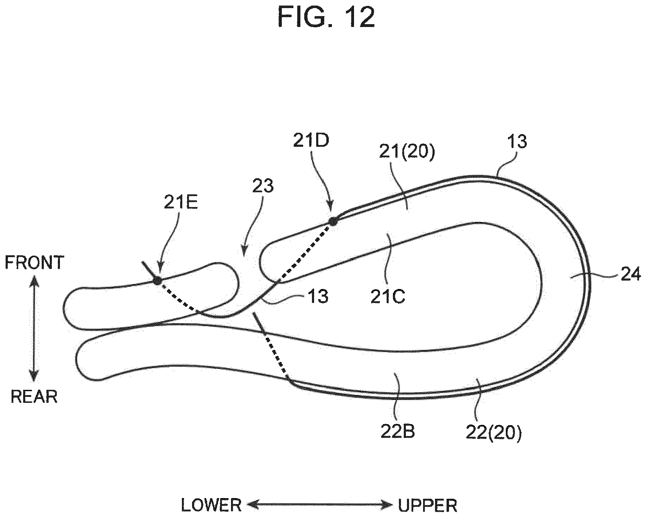

Next, the suture thread 13 goes around the body part 22B of the rear wall 22, the bottom portion 24 and the body part 21C of the front wall 21 in this order (FIG. 10). Then, the suture thread 13 is passed inward of the uterus 20 by causing the suture needle to penetrate a front wall portion 21D above the caesarean section wound portion 23 from outside of the uterus toward inside of the uterus (FIG. 11). Subsequently, the suture thread 13 is pulled out from the front wall 21 side to outside of the uterus by causing the suture needle to penetrate the front wall portion 21E below the caesarean section wound portion 23 from inside of the uterus toward outside of the uterus (FIG. 12). In this procedure, the right part of the uterus 20 is also ligated with the suture thread 13 as shown in FIG. 4. Finally, the caesarean section wound portion 23 is sutured.

Next, one example of compression suture of the uterine body lower portion will be described. This is a method to be applied to a disease with bleeding observed from the uterine body lower portion including a placenta previa. By this method, hemostasis is performed through suppression of blood flow to the body lower portion 20A by suturing the front wall 21 and the rear wall 22 like bringing them together in the body lower portion 20A of the uterus 20 (part below the caesarean section wound portion 23). Here, since there are many blood vessels in the neighboring tissues of the body lower portion 20A of the uterus 20, when a suture needle with a pointed puncture portion is used, a risk of damaging the blood vessels will increase. Meanwhile, in the present embodiment, the suture needle for uterine hemostatic compression 1 including the suture needle 10 with the puncture portion 15 formed in a spherical surface is used as described above. Therefore, suturing the body lower portion 20A of the uterus 20 can also be performed safely.

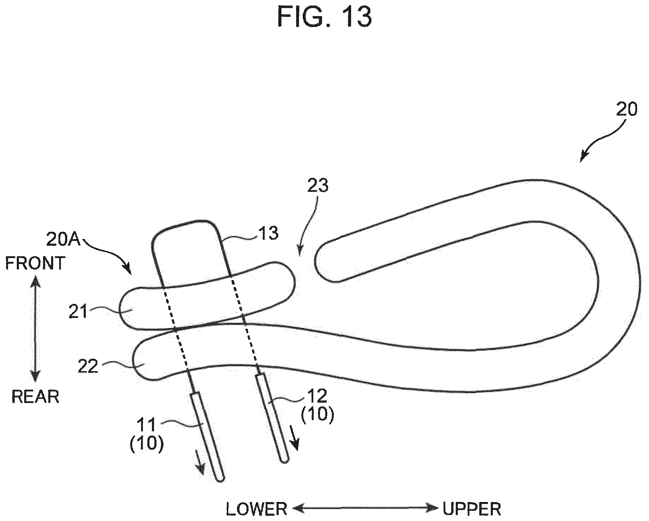

Specifically, the operator grips the first suture needle 11 and the second suture needle 12 with a needle holder, and brings the front wall 21 and the rear wall 22 close to each other by holding, pressing, and contracting the body lower portion 20A of the uterus 20 with a hand. Then, in order to confirm that the first suture needle 11 and the second suture needle 12 have penetrated the front wall 21 and the rear wall 22, the operator holds a finger in advance on the rear wall 22 side. Then, as shown in FIG. 13, the operator thrusts the first suture needle 11 and the second suture needle 12 at the same time from the front wall 21 side so as to penetrate the front wall 21 and the rear wall 22 at a stretch toward the finger held on the rear wall 22 side. Then the operator passes the suture thread 13 through the front wall 21 and the rear wall 22.

In this way, the operator causes the first suture needle 11 and the second suture needle 12 to penetrate the body lower portion 20A of the uterus 20, and ligates the suture thread 13 on the rear wall 22 side. As a result, the front wall 21 and the rear wall 22 of the uterus 20 are in complete contact with each other, the blood flow to the body lower portion 20A of the uterus 20 is suppressed, and hemostasis can be performed. Such an operation is performed at two places on the right side and the left side of the uterus 20 in FIG. 4, and may be performed at three places including the middle portion.

Normally, in hemostasis of atonic bleeding, in order to prevent damage to blood vessels in the body lower portion 20A of the uterus 20, the manipulation as described above is performed in the upper part of the caesarean section wound portion 23, and the front wall 21 and the rear wall 22 are brought into close contact. Meanwhile, in the present embodiment, by using the extremely dull suture needle 10 having the puncture portion 15 with a spherical surface, it is possible to thrust the suture needle 10 into tissues while preventing damage to blood vessels even in the body lower portion 20A of the uterus 20. Therefore, it is possible to safely suture even in the body lower portion 20A of the uterus 20 where there are many blood vessels. As described above, by using the two suture needles 10 connected by one suture thread 13, the uterus 20 can be sutured more easily and quickly than when one suture needle is used. This allows contribution to quick operational medical services.

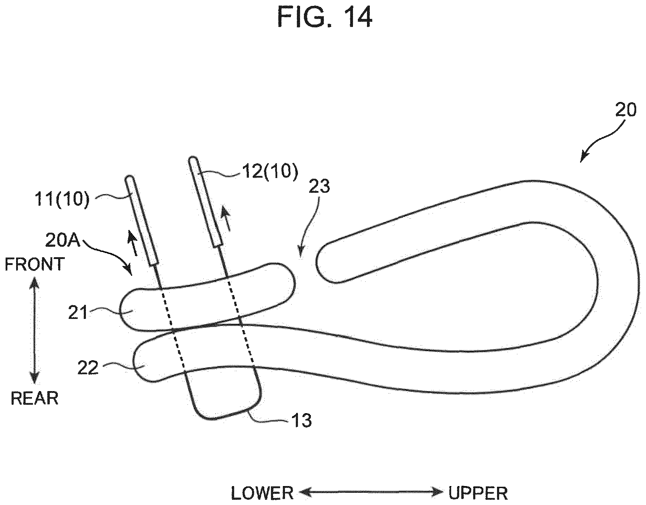

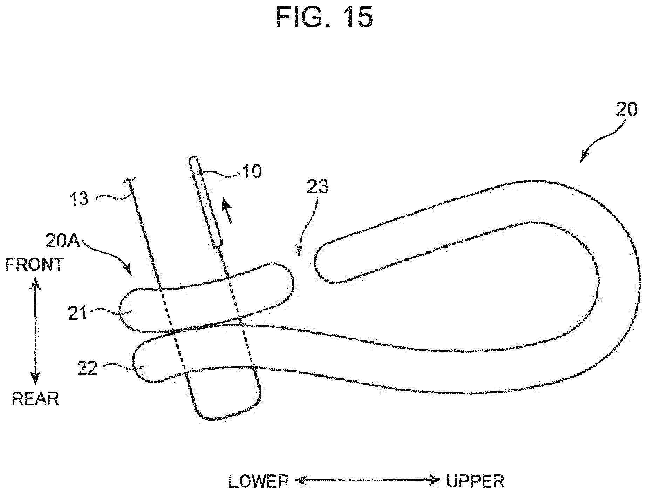

Note that the method of suturing the body lower portion 20A of the uterus 20 is not limited to the aspect shown in FIG. 13, but the following modified method is also possible. As shown in FIG. 14, the operator may cause the first suture needle 11 and the second suture needle 12 to penetrate the uterus 20 from the rear wall 22 side and ligate the suture thread 13 on the front wall 21 side. Also, the method of suturing the body lower portion 20A of the uterus 20 is not limited to using the two suture needles 10. As shown in FIG. 15, after one suture needle 10 performs penetration from the front wall 21 toward the rear wall 22, the one suture needle 10 may perform penetration from the rear wall 22 toward the front wall 21, and the suture thread 13 may be ligated on the front wall 21 side. Conversely, after one suture needle 10 performs penetration from the rear wall 22 toward the front wall 21, the one suture needle 10 may perform penetration from the front wall 21 toward the rear wall 22, and the suture thread 13 may be ligated on the rear wall 22 side.

### [Operational effects]

Next, features and operational effects of the suture needle for uterine hemostatic compression 1 will be described.

The suture needle for uterine hemostatic compression 1 is used for hemostatic compression of the body lower portion 20A of the uterus 20. The suture needle for uterine hemostatic compression 1 includes the suture needle 10 and the suture thread 13. The suture needle 10 includes the body portion 14 extending linearly in the lengthwise direction D1 and the puncture portion 15 provided at a tip of the body portion 14 in the lengthwise direction D1 to be thrust into the uterus 20. The suture thread 13 attached to the base end portion 17 that is on an opposite side of the puncture portion 15 in the suture needle 10. The puncture portion 15 is formed as a spherical surface 15A that bulges toward a tip side of the lengthwise direction D1 and has the radius R1 in the connection portion with the body portion 14 substantially identical to a radius R2 of the body portion 14.

The suture needle for uterine hemostatic compression 1 makes it possible to pass the suture thread 13 by thrusting the puncture portion 15 of the suture needle 10 into the body lower portion 20A of the uterus 20 for penetration, and to perform hemostatic compression on the body lower portion 20A of the uterus 20 by ligating the suture thread 13. Here, since the puncture portion 15 is formed as the spherical surface 15A, it is possible to prevent many blood vessels existing in the body lower portion 20A of the uterus 20 and neighboring peripheral organs from being damaged by the puncture portion 15 when the suture needle 10 performs penetration. Moreover, since the body portion 14 of the suture needle 10 has a shape extending linearly in the lengthwise direction D1, it is possible to cause the body portion 14 of the suture needle 10 to travel along a path through which the puncture portion 15 passes within the uterus. Therefore, unlike the suture needle 10 with the body portion 14 having a curved shape, it is possible to cause the suture needle 10 to perform straight penetration. Therefore, it is possible for the operator to securely suture a part to be sutured, and to minimize a risk of damaging blood vessels of neighboring tissues of the uterus 20 and peripheral organs by mistake. Therefore, the suture needle for uterine hemostatic compression 1 enables an operation to perform hemostatic compression of the body lower portion 20A of the uterus 20 to be safely performed.

In the suture needle for uterine hemostatic compression 1, the width W1 of the puncture portion 15 is 1 mm or more. Since this will significantly increase thrusting resistance of the suture needle 10, it is considered that the damage to blood vessels in the body lower portion 20A of the uterus 20 can be suppressed more effectively during an operation. Even when the operator touches the puncture portion 15 with a finger during the operation, possibility of damaging the finger will decrease.

In the suture needle for uterine hemostatic compression 1, the outer peripheral surface 14A of the body portion 14 is formed so as to follow the tangent line S1 at the terminal 15B of the spherical surface 15A. As a result, the connection portion between the puncture portion 15 and the body portion 14 becomes smoother, making it easier to thrust the suture needle 10 into the uterus 20.

In the suture needle for uterine hemostatic compression 1, the center C1 of the spherical surface 15A is positioned at the terminal 15B of the spherical surface 15A. The body portion 14 is formed so as to extend from the terminal 15B of the spherical surface 15A toward the base end portion 17 with a constant width in the lengthwise direction D1. This makes it possible to more effectively prevent bleeding that can be caused by a difference in the width of the body portion 14 in the lengthwise direction when the suture needle 10 penetrates straight the body lower portion 20A of the uterus 20.

In the suture needle for uterine hemostatic compression 1, the suture needle 10 includes the first suture needle 11 and the second suture needle 12. One end 13A of the suture thread 13 is attached to the base end portion 17 of the first suture needle 11, and the other end 13B of the suture thread 13 is attached to the base end portion 17 of the second suture needle 12. Accordingly, by using the two suture needles 10, the uterus 20 can be sutured more easily and quickly than when one suture needle is used. In addition, it becomes possible to widen variation of the suturing method, and to perform appropriate suturing for various cases.

In the suture needle for uterine hemostatic compression 1, the length L1 of the suture needle 10 is 60 mm or more. In hemostasis of atonic bleeding, the suture needle 10 penetrates at a stroke both the front wall 21 and the rear wall 22 of the body lower portion 20A of the uterus 20, then the operator confirms that the suture needle 10 has penetrated the uterine wall by touching the puncture portion 15 of the suture needle 10 with a finger. Considering the thicknesses T1 and T2 of the uterine walls being about 25 mm and the length of the portion where the operator grasps the suture needle 10 and the portion protruding from the uterine wall, the length L1 of the suture needle 10 needs to be 60 mm or more. However, the suture needle for uterine hemostatic compression of the present invention is not limited to one with this length, but may be one with the length L1 of less than 60 mm.

### (Other embodiments)

Next, the suture needle for uterine hemostatic compression according to other embodiments of the present invention will be described.

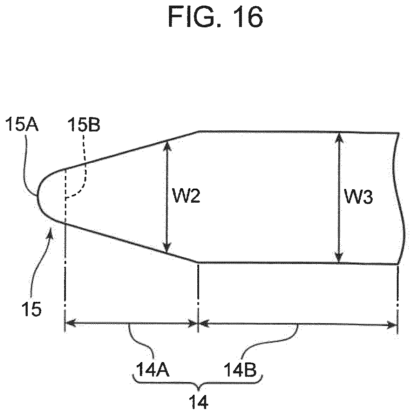

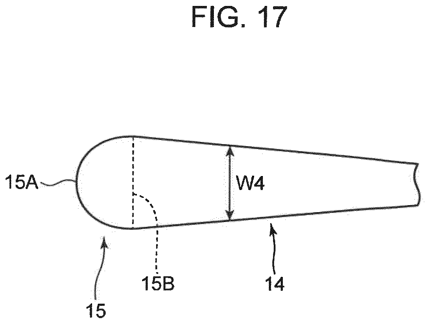

The first embodiment has described a case where the body portion 14 extends with a constant width from the terminal 15B of the spherical surface 15A toward the base end portion 17, but the present invention is not limited to this case. As shown in FIG. 16, the body portion 14 may have a flaring first body portion 14A with a width W2 gradually increasing as going away from the terminal 15B of the spherical surface 15A and a second body portion 14B extending with a constant width W3. The body portion 14 may have a shape in which the first body portion 14A and the second body portion 14B are connected to each other. Furthermore, as shown in FIG. 17, the body portion 14 may have a tapered shape in which a width W4 gradually decreases as going away from the terminal 15B of the spherical surface 15A.

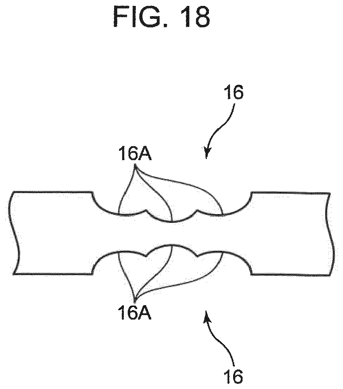

Furthermore, as shown in FIG. 18, a plurality of (three in FIG. 18) depressed portions 16A may be formed in a bottom portion of the grip portion 16. This makes it possible to make the structure easier for the operator to hold.

Note that the outline of the above embodiments is as follows.

A suture needle for uterine hemostatic compression according to the present embodiment includes at least one suture needle and a suture thread. The at least one suture needel includes a body portion extending linearly in a lengthwise direction and a puncture portion provided at a tip of the body portion in the lengthwise direction to be thrust into a uterus. The suture thread is attached to a base end portion that is on an opposite side of the puncture portion in the suture needle. The puncture portion is formed as a spherical surface that bulges toward a tip side of the lengthwise direction, the spherical surface having a radius in a connection portion with the body portion substantially identical to a radius of the body portion.

The suture needle for uterine hemostatic compression makes it possible to pass the suture thread by thrusting the puncture portion of the suture needle into the body lower portion of the uterus for penetration, and to ligate the body lower portion of the uterus by using the suture thread. Here, there are many blood vessels in the neighboring tissues of the body lower portion of the uterus. Therefore, when the suture needle is thrust into the body lower portion of the uterus, the suture needle may damage the blood vessels of the neighboring tissues. Also, since the body lower portion of the uterus is close to peripheral organs such as the bladder and the rectum, suturing the body lower portion of the uterus has a high risk of organ damage. Meanwhile, in the suture needle for uterine hemostatic compression, the puncture portion is formed as the spherical surface. Therefore, when the suture needle performs penetration, it is possible to prevent many blood vessels in the body lower portion of the uterus from being damaged by the puncture portion, and to reduce the risk of damage to peripheral organs. Moreover, since the body portion of the suture needle has a shape extending linearly in the lengthwise direction, it is possible to cause the body portion of the suture needle to travel along a path through which the puncture portion passes within the uterus. Therefore, unlike the suture needle with the body portion having a curved shape, it is possible to cause the suture needle to perform straight penetration. This makes it possible for the operator to securely suture a part to be sutured, and to minimize a risk of damaging blood vessels of neighboring tissues of the uterus by mistake. Therefore, the suture needle for uterine hemostatic compression enables the operation to perform hemostatic compression of the uterus to be safely performed.

Note that the radius in the connection portion between the spherical surface and the body portion being substantially identical is not limited to a case of completely identical. A shift of a level that can prevent damage to the blood vessels when the suture needle penetrates the uterus as described above is also permitted.

In the suture needle for uterine hemostatic compression, a width of the puncture portion may be 1 mm or more.

The present inventors and the like have conducted intensive studies and found that when the width of the puncture portion is 1 mm or more, the thrusting resistance of the suture needle (force required to thrust the suture needle into an object assuming the uterus) becomes much larger than when the width of the puncture portion is less than 1 mm. Therefore, by setting the width of the puncture portion at 1 mm or more, it is considered that it will be difficult for the suture needle to penetrate tissues with different hardness and elasticity such as blood vessels. It is considered that this makes it possible to more effectively suppress damage to blood vessels that cannot be visually observed while the suture needle travels in the tissues for sewing. Also, even when the operator touches the puncture portion with a finger, the possibility of damaging the finger will decrease. As a result of various studies from such a viewpoint, the width of the puncture portion is preferably 1.05 mm or more, more preferably 1.1 mm or more, and even more preferably 1.15 mm or more.

In the suture needle for uterine hemostatic compression, an outer peripheral surface of the body portion may be formed to follow a tangent line at a terminal of the spherical surface.

With this configuration, the connection portion between the puncture portion and the body portion becomes smoother, making it easier to thrust the suture needle into the uterus.

In the suture needle for uterine hemostatic compression, a center of the spherical surface may be positioned at the terminal of the spherical surface. The body portion may be formed to extend with a constant width in the lengthwise direction from the terminal of the spherical surface toward the base end portion.

This configuration makes it possible to more effectively prevent bleeding that can be caused by a difference in the width of the body portion in the lengthwise direction when the suture needle penetrates straight the uterus than the configuration with a shape in which the width of the body portion increases from the terminal of the spherical surface toward the base end portion.

In the suture needle for uterine hemostatic compression, the suture needle may include a first suture needle and a second suture needle. One end of the suture thread may be attached to the base end portion of the first suture needle. The other end of the suture thread may be attached to the base end portion of the second suture needle.

This configuration can further widen the range of variations of the suturing method by using two suture needles. This makes it possible to perform appropriate suturing for various cases

In the suture needle for uterine hemostatic compression, a length of the suture needle may be 60 mm or more.

In hemostasis of atonic bleeding, the suture needle penetrates at a stroke both the front wall and the rear wall of the body lower portion of the uterus, then the operator confirms that the suture needle has penetrated the uterine wall by touching the puncture portion of the suture needle with a finger. Here, the thickness of each uterine wall is about 25 mm, and when a length of a portion in which the operator holds the suture needle and a length of a portion protruding from the uterine wall are taken into consideration, the length of the suture needle is preferably 60 mm or more, more preferably 80 mm or more, and even more preferably 100 mm or more.

### Examples

### (Test method)

The thrusting resistance of the suture needles of No.1 to No.6 shown in Table 1 below was measured. No.1 to No.4 are suture needles of the structure shown in FIG. 2, No.5 is a suture needle of the structure shown in FIG. 16, and No.6 is a suture needle of the structure called a round needle with a sharply pointed puncture portion. No.1 to No.5 are examples of the present invention, and No.6 is a comparative example.

The width W1 (mm) of the needle tip, the width W2 (mm) of the base end portion of the needle and the radius R1 (mm) of the puncture portion in each suture needle are as in Table 1. The radius R1 of the puncture portion is a value obtained by magnifying the tip of each suture needle and measuring the radius of the spherical portion of the needle tip. Note that in the comparative example of No. 6, since the puncture portion of the needle tip is sharply pointed, both the width W1 and the radius R1 of the needle tip were unmeasurable. A surgical glove (thickness 0.24 mm), silicone rubbers (thickness 0.05 mm, 0.5 mm) and a pig liver (thickness 5 mm) were used to thrust the suture needle.

The surgical glove, the silicone rubbers and the pig liver were each set on a dedicated jig, which was mounted on a tension tester. Then, magnitude of force when each suture needle of No.1 to No.6 is thrust into each object was measured as thrusting resistance (N). Measurement results are as shown in Table 1 below.

**[Table 1]**

| Sample | Suture needle | | | Thrusting resistance (N) | | | | Example/Comparative example |
|---|---|---|---|---|---|---|---|---|
| | Width of needle tip W1 (mm) | Width of base end portion of needle W2 (mm) | Radius R1 (mm) | Surgical glove (Thickness 0.24 mm) | Silicone rubber (Thickness 0.05 mm) | Silicone rubber (Thickness 0.5 mm) | Liver (Thickness 5 mm) | |
| No.1 | 1.153 | 1.158 | 0.553 | - | 1.10±0.23 | 10.53±0.48 | 0.64±0.16 | Example |
| No.2 | 0.773 | 0.775 | 0.375 | 2.82±0.16 | 0.53±0.23 | 6.28±0.70 | 032±0.10 | Example |
| No.3 | 0.618 | 0.62 | 0.299 | 2.33±0.12 | 0.49±0.16 | 5.26±1.04 | 0.27±0.12 | Example |
| No.4 | 0.353 | 0.356 | 0.18 | 1.51±0.12 | 0.28±0.12 | 4.63±0.92 | 0.17±0.05 | Example |
| No.5 | 0.244 | 1.141 | 0.123 | 1.95±0.16 | 0.1 or less | 2.67±0.54 | 0.1 or less | Example |
| No.6 | - | 1.08 | - | 0.26±0.07 | 0.1 or less | 0.1 or less | 0.1 or less | Comparative example |

### (Consideration)

As shown in Table 1, in the examples of No.1 to No.5, the thrusting resistance (N) was conspicuously larger than the thrusting resistance of the comparative example of No.6. This is considered because the puncture portion in the suture needle is formed as a spherical surface, making it harder for the suture needle to be thrust into each object. In No.1 with the width W1 of the needle tip of 1 mm or more and the radius R1 of 0.5 mm or more, the thrusting resistance became much larger than in No.2 to No. 5 with the width W1 of less than 1 mm and the radius R1 of 0.5 mm or less. This shows that the hardness for the suture needle to be thrust greatly changes with the needle tip width of 1 mm and the radius of 0.5 mm as a boundary. Note that when the suture needle of No.1 was thrust into the surgical glove, the needle was not thrust and the glove was broken, and thus measurement of the thrusting resistance was impossible.

## Claims

1. A suture needle for uterine hemostatic compression comprising:
at least one suture needle including a body portion extending linearly in a lengthwise direction and a puncture portion provided at a tip of the body portion in the lengthwise direction to be thrust into a uterus; and
a suture thread attached to a base end portion that is on an opposite side of the puncture portion in the suture needle,
wherein the puncture portion is formed as a spherical surface that bulges toward a tip side of the lengthwise direction, the spherical surface having a radius in a connection portion with the body portion substantially identical to a radius of the body portion.

2. The suture needle for uterine hemostatic compression according to claim 1, wherein a width of the puncture portion is 1 mm or more.

3. The suture needle for uterine hemostatic compression according to claim 1 or 2, wherein an outer peripheral surface of the body portion is formed to follow a tangent line at a terminal of the spherical surface.

4. The suture needle for uterine hemostatic compression according to claim 3, wherein
a center of the spherical surface is positioned at the terminal of the spherical surface, and
the body portion is formed to extend with a constant width in the lengthwise direction from the terminal of the spherical surface toward the base end portion.

5. The suture needle for uterine hemostatic compression according to any one of claims 1 to 4, wherein
the suture needle includes a first suture needle and a second suture needle,
one end of the suture thread is attached to the base end portion of the first suture needle, and
the other end of the suture thread is attached to the base end portion of the second suture needle.

6. The suture needle for uterine hemostatic compression according to any one of claims 1 to 5, wherein a length of the suture needle is 60 mm or more.
